# EUROPEAN PATENT SPECIFICATION

(11) **EP 2 762 487 B1**
(45) Date of publication and mention of the grant of the patent: **27.12.2017**
(21) Application number: 12834743.2
(22) Date of filing: 28.09.2012
(51) Int. Cl.: C07K 14/415, G01N 33/68, A61K 38/08

(54) **IMMUNOGENIC GLUTEN PEPTIDES AND USES THEREOF**
IMMUNOGENE GLUTENPEPTIDE UND IHRE VERWENDUNG
PEPTIDE IMMUNOGÈNE DU GLUTEN ET SES APPLICATIONS

(30) Priority: 29.09.2011 ES 201131576
(43) Date of publication of application: 06.08.2014
(73) Proprietor: Universidad De Valladolid, 47002 Valladolid (ES)
(72) Inventor: BERNARDO ORDIZ, David, 47002 Valladolid (ES); GARROTE ADRADOS, José Antonio, 47002 Valladolid (ES); BLANCO QUIRÓS, Alfredo Ramón, 47002 Valladolid (ES); ARRANZ SANZ, Eduardo, 47002 Valladolid (ES); CEBOLLA RAMIREZ, Ángel, 47002 Valladolid (ES)
(74) Representative: Pons
(86) International application number: PCT/ES2012/070673
(87) International publication number: WO 2013/045737

(56) References cited:
- EP-A1- 0 905 518
- EP-A2- 0 821 238
- WO-A1-2010/060155
- WO-A2-2005/105129
- US-A1- 2006 240 475
- US-A1- 2006 286 601
- VADER W ET AL: "THE GLUTEN RESPONSE IN CHILDREN WITH CELIAC DISEASE IS DIRECTED TOWARD MULTIPLE GLIADIN AND GLUTENIN PEPTIDES", GASTROENTEROLOGY, ELSEVIER, PHILADELPHIA, PA, vol. 122, no. 7, 1 June 2000 (2000-06-01), pages 1729-1737, XP009013823, ISSN: 0016-5085, DOI: 10.1053/GAST.2002.33606
- SARA VALLEJO-DIEZ ET AL: "Detection of Specific IgA Antibodies against a Novel Deamidated 8-Mer Gliadin Peptide in Blood Plasma Samples from Celiac Patients", PLOS ONE, vol. 8, no. 11, 22 November 2013 (2013-11-22), page e80982, XP055173113, DOI: 10.1371/journal.pone.0080982
- CAMARCA A. ET AL.: 'Intestinal T Cell Responses to Gluten Peptides Are Largely Heterogeneous: Implications for a Peptide-Based Therapy in Celiac Disease.' THE JOURNAL OF IMMUNOLOGY vol. 182, no. 7, 2009, pages 4158 - 4166, XP055070215

### SEQUENCE LISTING

<110> Universidad de Valladolid
<120> IMMUNOGENIC GLUTEN PEPTIDE AND APPLICATIONS THEREOF
<130> ES2080.8
<160> 9
<170> PatentIn version 3.5
<210> 1
   <211> 8
   <212> PRT
   <213> Triticum aestivum
<220>
   <221> VARIANT
   <222> (4)..(4)
   <223> Replaceable for Glu
<220>
   <221> VARIANT
   <222> (6)..(7)
   <223> Replaceable for Glu
<400> 1
   Phe Pro Leu Gln Pro Gln Gln Pro
   1               5
<210> 2
   <211> 8
   <212> PRT
   <213> Triticum aestivum
<400> 2
   Phe Pro Leu Gln Pro Gln Gln Pro
   1 5
<210> 3
   <211> 8
   <212> PRT
   <213> Artificial sequence
<220>
   <223> Deamidated peptide 8-mer
<400> 3
   Phe Pro Leu Gln Pro Glu Gln Pro
   1 5
<210> 4
   <211> 19
   <212> PRT
   <213> Triticum monococcum
<400> 4
   Leu Gly Gln Gln Gln Pro Phe Pro Pro Gln Gln Pro Tyr Pro Gln Pro
   1 5 10 15
   Gln Pro Phe
<210> 5
   <211> 33
   <212> PRT
   <213> Artificial sequence
<220>
   <223> Gluten peptide 33-mer
<400> 400
<210> 6
   <211> 9
   <212> PRT
   <213> Artificial sequence
<220>
   <223> SEQ ID NO: 3 with a lisine in C-terminus
<400> 6
   Phe Pro Leu Gln Pro Glu Gln Pro Lys
   1               5
<210> 7
   <211> 9
   <212> PRT
   <213> Artificial sequence
<220>
   <223> SEQ ID NO: 2 with a lisine in C-terminus
<400> 7
   Phe Pro Leu Gln Pro Gln Gln Pro Lys
   1               5
<210> 8
   <211> 15
   <212> PRT
   <213> Triticum aestivum
<400> 8
   Pro Phe Ile Gln Pro Ser Leu Gln Gln Gln Leu Asn Pro Cys Lys
   1               5                   10                  15
<210> 9
   <211> 18
   <212> PRT
   <213> Triticum aestivum
<400> 9
   Val Phe Leu Gln Gln Gln Cys Ser Pro Val Ala Met Pro Gln Ser Leu
   1               5                   10                  15
   Ala Arg

## Description

The present invention is placed on the field of health and food, specifically within the immunogenic gluten peptides useful for the diagnosis and/or monitoring of celiac disease in subjects as well as for the detection of gluten in foods.

### BACKGROUND OF THE INVENTION

Celiac disease is a gliadin-induced enteropathy (prolamin from wheat) and other related cereal prolamins as secalin (rye), hordein (barley) and some avenins (oats) in genetically predisposed individuals (HLA-DQ2/DQ8).
Currently, the immunopathogenesis of celiac disease is explained by a model that includes two different signals. On one hand, some of the gliadin peptides that would be generated during the gastrointestinal digestion, such as 19-mer peptide, play a fundamental role in the development of celiac disease by triggering an innate immune response (Jabri B. and Sollid LD., 2006, Nat Clin Pract Gastroenterol Hepatol;. 3(9):516-525) through DQ2 independent mechanisms leading to the production of IL-15 by epithelial cells. The result is the disruption of the epithelial barrier and the induction of apoptosis in enterocytes. Furthermore, other immunodominant peptides, such as 33-mer peptide (Shan L., et al., 2002, Science;. 297(5590):2275-2279), transform some of its glutamine residues into glutamate (deamidation) by action of tissue transglutaminase (tTG) and can reach the lamina propria where they are presented by dendritic cells to specific T lymphocytes restricted to molecules HLA-DQ2/DQ8. Then the inflammation that occurs fits into a type I cytokine profile mediated by IFNy, and the damage is characterized by massive infiltration of intraepithelial lymphocytes, cryptic hyperplasia and villous atrophy.
Today, the only treatment for celiac disease is to maintain a lifelong gluten-free diet, which often leads to complete remission of the disease. Such treatment is well tolerated by celiac patients and represents a clear improvement in their health and quality of life. However, following rigorously a gluten-free diet is very difficult due to, for example, the presence of trace contaminants in gluten-free food, the high price of this type of food or social pressure to which these patients are subjected, especially younger, to consume gluten caused by eating habits prevailing in the West. For all these reasons, in recent years alternative therapies to the gluten-free diet have been investigated which try to combat the disease in different ways: reducing exposure to gluten (for example by enzymatic degradation of toxic fractions of cereals), inhibiting the intestinal permeability or modulating the immune response (for example, through the development of vaccines based on peptides from gluten, or the inhibition of tTG).

The cancellation of the toxic and immunogenic activity of gluten by enzymatic degradation is very atractive as an alternative oral therapy. In this sense, the detoxifying activity of various enzymes has been tested, such as prolyl endopeptidase or a specific glutamine endoprotease. Another enzyme source for the hydrolysis of gluten is probiotic bacteria. However, it is clear that the success of such therapies is linked to a better and more complete characterization of the peptides that make up the toxic fraction of gluten.

Another aspect of celiac disease that is being developed is the diagnosis. Currently, the only test accepted by the medical community to definitively diagnose a celiac patient is a histopathological analysis of biopsies from the small intestine, which should show the typical morphological abnormalities. However, there are different serological tests that are less invasive than biopsy, and are indicated as a first step in diagnosing the disease. The most sensitive and specific serological test is the detection of IgA type autoantibodies against endomysium (EMA) or against tTG. The development of tests to detect the presence of antibodies against deamidated gluten toxic peptides has also demonstrated to be a useful tool in the diagnosis of disease (Tack GJ., 2010, Nature Reviews Gastroenterology and Hepatology; 7:204-213).

To progress in the development of better diagnosis and treatment of celiac disease is essential to study its pathogenesis, in which environmental, genetic and immunological factors interact in a complex way. The essential environmental factors for the development of the disease are of food type: wheat gluten and other related proteins induce innate and adaptive immune responses that cause damage to the mucosa of the small intestine. Gluten comprises a set of over 100 storage proteins found in wheat seeds. Depending on the level of solubility, gluten is divided into gliadins and glutenins, both involved in celiac disease. There are homologues of these proteins in barley, rye and some oat varieties, which explain that these cereals can also cause the disease. It has been proposed that there are peptides from gluten, and related proteins from barley, rye and oat, that are not fully digested by the gastro-intestinal and pancreatic enzymes, which causes that, under certain conditions, they can penetrate the lamina propria of the intestine. Furthermore, it has been speculated that certain stress factors, such as viral infections, can affect intestinal permeability, favouring the appearance of celiac disease.

Gluten peptides resistant to digestion are rich in proline and glutamine, since most proteases do not cut residues adjacent to proline, and glutamine is not a preferential residue for any of the bowel endoproteases. Therefore, peptides with length sufficient to trigger an immune response are capable of avoiding the gastrointestinal digestion and reaching intact the epithelium. This is the case of the 33-mer peptide of the α-gliadin from wheat, which is resistant to proteases and has been considered as the primary initiator of the inflammatory response to gluten in celiac patients. Thus, it has been shown by *in vitro* assays that peptide 33-mer is the more bioactive gluten peptide (immunodominant) that is recognized by T cells from celiac donor HLA-DQ2 +, although it is not the only peptide that shows such activity.

The identification of the peptides from gluten which trigger response by intestinal T cells is crucial, among other things, for the search of alternative therapies to gluten-free diet. The literature contains some gluten peptides with demonstrated immunotoxic activity (Tye-Din J., et al., 2010, Science Translational Medicine; 2(41):1-14; Camarca A., et al., 2009, The Journal of Immunology; 189:4158-4166; Vader, et al., 2002, Gastroenterology; 122:1729-1737), most of which belong to the families α or γ-gliadin, followed by glutenins and less frequently to the ω-gliadin. The toxic gluten peptides are applicable in diagnostic and/or treatment methods for celiac patients (WO03066079, EP0905518, US5817523, WO9727217, US20080318852, US20090156490 and US20060240475).

Another application derived from the identification of a gluten peptide with immunotoxic activity consists in measuring the toxic fraction of the gluten in food. The most established techniques for the control of gluten in food are ELISA assays, PCR, Western Blot, mass spectrometry, chromatography and immunochromatography strips. Among these, enzyme immunosorbent assays (ELISA) based on monoclonal antibodies against specific epitopes are the techniques which meet the most desirable characteristics of simplicity, sensitivity and economy. Techniques that use biosensors and technology lab-on-a-chip are newer and still in development.

For the detection of gluten by ELISA different antibodies recognizing different epitopes of gluten have been developed. It has been reported that not only gliadin peptides are toxic to celiacs, but glutenin peptides are toxic too. This is the reason why it is desirable that a method for detecting gluten toxicity in food is capable of measuring peptides present in both types of proteins. Monoclonal antibodies R5 (Sorell L., et al., 1998, FEBS Lett; 439:46-50), G12 (Moron B., et al, 2008, PLoS ONE; 3:405-414), 401.21 (Skerritt J., et al., 1990, J. Agric. Food Chem.; 38:1771-1778) and PN3 (Bermudo Redondo, et al., 2005, Analytica Chimica Acta; 551:105-114) are specific for different fractions of gluten and thus are applied to the detection of it in food. Also, the patents WO2006004394, WO2006051145, ES2142720, GB2207921 and AU611921 propose procedures based on ELISA technique for the detection of gluten in food.

Finally, the methodology used to identify peptides from gluten causing immunotoxicity often includes an *in vitro* digestion step with proteases of a gluten solution. Algorithms have sometimes been used to predict patterns of gluten deamidation by tTG, or to generate the potential epitopes recognizable by intestinal T cells from known sequences of prolamins or glutenins. However, despite the existing idea that celiac patients digest gluten differently to healthy individuals, thus far no efforts have been made to characterize the peptides generated specifically in the intestine of celiac patients as a result of its proteolytic activity, being these of particular interest for the diagnosis and/or treatment of celiac disease in individuals, due to that their application in this regard would be a clear improvement in terms of specificity and efficiency over existing methods which are based on other immunogenic peptides.

### DESCRIPTION OF THE INVENTION

The present invention provides an isolated peptide of eight amino acids, hereafter "peptide of the invention" or "peptide 8-mer", which is generated naturally in the gut of celiac patients by the action of bacterial proteases present in their intestinal flora that degrade gluten gliadin. The inventors demonstrate that this peptide has immunogenic capacity, being able to stimulate immune system cells in culture both from celiac patients and non-celiac subjects. Therefore, and because the peptide of the invention is generated *in vivo* in the intestine of celiac patients and not in the intestine of healthy individuals or those with other diseases, said peptide is a highly specific marker for the diagnosis and/or monitoring of this disease, as well as an interesting therapeutic target for the development of compounds or compositions useful for the diagnosis, treatment and/or prevention of this pathological condition. Moreover, due to its immunogenicity, the peptide of the invention is also useful as a therapeutic agent. Furthermore, antibodies against the peptide of the invention are useful for the detection and/or quantification of the latter preferably in food, which allows knowing the gluten toxicity therein.
Therefore, a first aspect of the invention relates to an isolated peptide of SEQ ID NO: 1, "peptide of the invention" or "peptide 8-mer". As mentioned, this peptide has immunogenic activity and is produced by the proteolysis that gliadin from gluten suffers in celiac patients intestine although, as the examples of the present invention show, said peptide is not only part of the gliadin sequences but also of sequences from other proteins belonging to other cereal species that are toxic for celiacs, as glutenins, secalins and hordeins.
The peptide of the invention can exhibit variations, which are related to limited variations in their amino acid sequence that enable the maintenance of the functionality of the peptide, i. e. an isolated peptide of SEQ ID NO: 2 or SEQ ID NO: 3. The peptide of the invention can present modifications resulting from its enzymatic processing. Thus, because the immunogenic peptides from gluten may undergo a deamidation in the intestine of celiac subjects (substituting glutamate for glutamine) by the action of tissue transglutaminase (tTG), positions 4, 6 and/or 7 of SEQ ID NO: 1 , which in the native form of the peptide are glutamines (Gin), can be replaced by glutamate (Glu). Therefore, in an embodiment the sequence of the peptide of the invention is SEQ ID NO: 2, peptide (native) isolated in the examples of this invention from proteins of several species of cereals toxic for celiacs, as for example, but not limited, barley, rye and wheat. SEQ ID NO: 2 corresponds to SEQ ID NO: 1, in which the positions 4, 6 and 7 are Gin. In another embodiment the peptide of the invention is deamidated, preferably as a result of its processing by the tTG, even more preferably , the peptide sequence of the invention is SEQ ID NO: 3, sequence that corresponds to SEQ ID NO : 1 in which the positions 4 and 7 are Gln and position 6 is Glu.

The peptides of the present invention may be synthesized, for example, but without limitation, by chemical synthesis, recombinant DNA techniques, isolation from natural sources or by *in vitro* proteolysis. The peptide of the invention may be produced by recombinant techniques, not only directly but also as a fusion polypeptide together with a heterologous polypeptide, which may contain for example but without limitation, a signal sequence or other polypeptide having a cleavage site for protease, for example but without limitation, at the N-terminus of the mature polypeptide or protein.

*In vitro* diagnosis of celiac disease in an individual may be performed using the peptides of the invention by various methods. One of these methods may be, but not limited to, the detection of the intestinal proteolytic activity which leads to the release of the peptide from gliadin, or other related proteins as secalins, hordeins or glutenins, which could be made, for example, using the peptides of the invention modified with at least one chromogenic, fluorigenic or luminescent substrate, so that, when it is in contact with a biological sample isolated from intestine, color is generated only in the case of celiac patients due to the presence of specific enzymes associated with their intestinal flora capable of performing the release of the peptide by proteolytic hydrolysis. Therefore, in a more preferred embodiment, the peptides of the invention further comprise a chemical compound attached to its N- and/or C-terminal. Preferably, this chemical compound is biotin, a molecule with affinity for streptavidin. That chemical compound may be added, for example, synthetically to the peptide of the invention by techniques known in the state of the art. In another preferred embodiment, the chemical compound is a chromogenic, fluorigenic or luminescent compound. A "chromogenic compound" is one that produces color or pigment. Herein the chromogenic compound is preferably p-nitroanilide. A "fluorigenic compound" is one which fluoresces when excited at a certain wavelength, for example, but without limitation, 4-methylumbelliferyl derivatives or o- or p-halomethyl phenols. A "luminescent compound" is one that emits photons instead of providing visible color, therefore, is a compound that emits light returning from an electronically excited state to its original state. In the last term, bioluminescence, photoluminescence and chemiluminescence are included. An example of a luminescent compound is, but not limited to, the luciferin-luciferase.

In an even more preferred embodiment, the peptides of the invention also have a heterologous polypeptide attached to its N- and/or C-terminal. The binding of a peptide to another one can be done by known techniques for obtaining fusion proteins.
Another aspect of the invention relates to an isolated nucleotide sequence encoding the peptide of the invention, hereinafter "nucleotide sequence of the invention". Due to the degeneracy of the genetic code, in which several triplets of nucleotides lead to the same amino acid, there are many nucleotide sequences that give rise to the same amino acid sequence.

The terms "nucleotide sequence", "nucleic acid", "oligonucleotide" and "polynucleotide" are used interchangeably herein and refer to a polymeric form of nucleotides of any length which may or may not be chemically or biochemically modified. The terms are referred, therefore, to any polyribonucleotide or polydeoxyribonucleotide, both single-stranded and double-stranded. The nucleotide sequence of the invention can be produced artificially by conventional cloning o selection methods, or by sequencing. Said nucleotide sequence, in addition to the coding sequence, can contain other elements, such as, but not limited to, introns, non-coding sequences at the 5 'and/or 3' end, ribosome binding sites, or stabilizing sequences. These polynucleotides may additionally include sequences encoding additional amino acids which may be useful, for example, but without limitation, to enhance stability of the peptide generated from them or to allow a better purification thereof.

The peptides of the invention show antigenic nature and thus can be used to develop monoclonal or polyclonal antibodies that specifically bind to it, which can be done by various methods known in the state of art. Therefore, another aspect of the invention relates to an antibody against the peptide of the invention, hereinafter "antibody of the invention".
One method that may be performed to obtain the antibody of the invention comprises, for example, but without limitation, of immunizing animals with the peptide of the invention and the subsequent purification, for example from the sera, of the specific antibodies generated against it.
The term "antibody" refers to molecules from immunoglobulins or immunologically active portions of molecules from immunoglobulins, i.e., molecules that contain an antigen binding site which specifically binds to (immunoreacts with) the peptide of the invention. Examples of portions of immunologically active molecules from immunoglobulins include F(ab) and F(ab')2, which can be generated, for example, but without limitation, by treating the antibody with an enzyme such as pepsin or by genetic engineering techniques known in the state of art.
The antibody of the invention may be polyclonal (typically including different antibodies directed against different determinants or epitopes) or monoclonal (directed against a single determinant on the antigen). The term "monoclonal antibody" refers to a population of antibody molecules that contain only one species of an antigen binding site capable of immunoreacting with a particular antigen epitope. The monoclonal antibody may be altered biochemically or by genetic manipulation or may be synthetic. In these cases the antibody possibly lacks in its totality or in parts, of portions that are not necessary for the recognition of the peptide of the invention, and being replaced by other which provide additional advantageous properties to the antibody.

The antibody of the invention exhibits specificity for the peptide of the invention, which is useful in several applications, as for the detection and/or quantification of the peptide of the invention, preferably in food forming part of the protein from which it comes, so that it is possible to detect gluten toxicity therein; for detection and/or quantification of the peptide of the invention in a biological sample isolated from an individual, with the aim of making *in vitro* diagnosis and/or monitoring of celiac disease, or for blocking the peptide of the invention, inhibiting partially or totally its immunogenic activity, in a method of treatment of this disease.

To carry out this last application in human individuals is convenient that the antibody of the invention is a humanized antibody, since no anaphylactic response from the immune system is generated when the humanized antibody of the invention is administered to a human. Using recombinant DNA technology it is possible to construct a humanized monoclonal antibody linking a variable region or of antigenic recognition of the antibody of the invention to a human antibody framework. In most cases, humanized antibodies are human immunoglobulins (recipient antibody) in which residues from hypervariable regions of the recipient are replaced by residues from a hypervariable region of a non-human species (donor antibody) having the desired specificity, affinity and capacity.

The term "hypervariable region" refers to amino acid residues of an antibody which are responsible for antigen binding. The hypervariable region comprises amino acid residues from a "complementarity determining region" or "CDR" and/or those residues from a "hypervariable loop". Supporting residues, called "framework" or "FR" residues are those residues from other than the hypervariable region. In some cases, the framework residues (FR) of the human immunoglobulin are replaced by corresponding non-human residues. Furthermore, humanized antibodies may comprise residues that are not found in the recipient antibody or in the donor antibody. These modifications are made to further refine antibody function. In general, the humanized antibody will comprise substantially all of at least one, and typically two, variable domains, in which all or substantially all of the hypervariable loops correspond to those of a non-human immunoglobulin and all or substantially all of the FR regions are from a human immunoglobulin sequence. The humanized antibody will also comprise, optionally, at least a portion of an immunoglobulin constant region (Fc), in general of a human immunoglobulin. Various methods for obtaining humanized antibodies are known in the state of the art.

Therefore, in a preferred embodiment, the antibody of the invention is a monoclonal antibody, more preferably humanized, and may be recombinant, chimeric, synthetic, or any combination thereof.

A "recombinant antibody or polypeptide " (rAC) is an antibody which has been produced in a host cell transformed or transfected with the nucleic acid encoding the antibody of the invention or the peptide of the invention, or that produces the antibody of the invention or the peptide of the invention as a result of homologous recombination. Said host cell includes a cell in an "in vitro" cell culture as well as a cell in a host animal.

The antibody of the invention may be chimeric. Thus, a region of the heavy and/or light chain is identical with or homologous to corresponding sequences in antibodies derived from a particular species or belonging to a particular class or subclass of antibodies, while the remaining chain(s) is(are) identical or homologous, to corresponding sequences in antibodies derived from another species or belonging to another class or subclass of antibodies, as well as to fragments of such antibodies, so that they exhibit the desired biological activity.

Another aspect of the invention relates to a cell that expresses the antibody of the invention, hereinafter "cell of the invention". The cell of the invention is preferably a B lymphocyte or a hybridoma, where "hybridoma" is a hybrid cell line obtained by fusing a B lymphocyte producing the antibody of the invention with a myeloma cell line (cancerous B lymphocyte) which does not produces an immunoglobulin itself; thus, it is an immortal cell line capable of producing the monoclonal antibody of the invention, which can be recovered from the medium.

Another aspect of the invention relates to a composition, hereinafter "composition of the invention", which comprises the peptide, the nucleotide sequence, the antibody or the cell of the invention.

The composition of the invention may further comprise adjuvants, excipients and/or pharmaceutically acceptable carriers. The term "excipient" refers to a substance which aids in absorption of the elements of the composition of the invention, stabilizes these elements, activates or assists in the preparation of composition in the sense of giving it consistency or providing flavors that made it more pleasant. Thus, carriers may have the function of holding the ingredients together, such as the case of starches, sugars or celluloses, sweetening function, the dye function, the function of protecting the composition, for example, to isolate from the air and/or moisture, the function of filling a tablet, capsule or any other form of presentation, the disintegrating function to facilitate dissolution of the components and their absorption in the intestine, without excluding other excipients not mentioned in this paragraph. The "pharmaceutically acceptable carrier", as the excipient, is a substance used in the composition to dilute any of the components of the present invention included therein to a given volume or weight. The pharmaceutically acceptable carrier is an inert substance or with analogous action of any of the objects of the invention. The function of the carrier is to facilitate the incorporation of other elements, to allow a better dosage and administration or to give consistency and shape to the composition. The term "adjuvant" refers to an agent that has no antigenic effect itself, but can stimulate the immune system increasing its response to the composition of the invention. There are many known adjuvants in the state of the art, such as, without limitation, aluminum phosphate, aluminum hydroxide, agonist of toll-like receptors, cytokines, squalene, Freund's incomplete adjuvant or Freund's complete adjuvant. Therefore, the term "composition" also includes a "pharmaceutical composition" and within what is known as "vaccine".

Preferably, the composition of the invention comprises the peptide, the nucleotide sequence, the antibody or the cell of the invention in a therapeutically effective amount, where "therapeutically effective amount" is the amount of peptide, nucleotide sequence, antibody, or cell of the invention that produces the desired effect. The dosage to obtain a therapeutically effective amount depends on a variety of factors, such as age, weight, sex and tolerance of the individual.

The composition of the present invention may be formulated for administration in a variety of ways known in the state of the art. Examples of preparations include any solid (tablets, pills, capsules, granules, etc.) or liquid (solutions, suspensions or emulsions) composition for oral, topical or parenteral administration. The composition of the present invention may also be formulated in the form of liposomes or nanospheres, of sustained release formulations or of any other conventional release system.

Such composition and/or its formulations thereof may be administered to an animal, including a mammal, and therefore to the man, in a variety of ways including, without limitation, parenteral, intraperitoneal, intravenous, intradermal, intraspinal, intrastromal, intraarticular, intrathecal, intralesional, intraarterial, intramuscular, intranasal, intracranial, subcutaneous, intracapsular, topical, transdermal patch, or rectally, by the administration of a suppository, percutaneous, nasal spray, surgical implant, internal surgical paint, infusion pump or via catheter.

As mentioned above, the immunogenic peptide of the invention is produced naturally in the gut of patients with celiac disease by the action of bacterial proteases in their intestinal flora that degrade gliadin from gluten, or secalin, hordein or glutenin, so that its use as a therapeutic target is interesting to identify compounds or compositions which serve for the diagnosis, prevention and/or treatment of celiac disease. Thus, another aspect of the invention relates to the use of the peptide or the nucleotide sequence of the invention for the identification or design of compounds or compositions for the diagnosis, prevention and/or treatment of celiac disease.

In a method for identifying or designing compounds or compositions mentioned in the preceding paragraph by using the peptide or the nucleotide sequence of the invention, such compounds or compositions could be classified as useful for the diagnosis of celiac disease when they are capable of specifically and selectively binding to the peptide of the invention, whether or not interfere with its biological activity or alter their structure. Similarly, in said method these compounds or compositions may be classified as useful for the prevention and/or treatment of celiac disease when they are capable of altering the biological activity of the peptide of the invention, thereby reducing or inhibiting completely the toxicity or immunogenicity.

Another aspect of the invention relates to the use of the antibody or the cell of the invention for the detection and/or quantification of the peptide of the invention. This application of the antibody and the cell of the invention is useful, not only for the *in vitro* diagnosis and/or monitoring of celiac disease in an individual, but also to determine the presence and/or amount of gluten in the foods, thus allowing to select those foods suitable for consumption by patients with celiac disease. Therefore, in a preferred embodiment, the detection and/or quantification of the peptide of the invention is performed in foods such as, but not limit to, cereals.

The advantage of detecting the peptide of the invention over other gluten peptides that have also been identified as immunogenic is that, as it is shown in the examples of the present invention, the peptide of the invention is widely distributed in various kinds of toxic cereals, including but not limited to, barley, rye and wheat (both wild and cultivated), and is also present not only in prolamins (gliadins, hordeins and secalins) but also in a glutenin with low molecular weight. Said detection and/or quantification can be performed by immunoassays, for example, but not limited to, by Western blot, immunoprecipitation, protein arrays, immunofluorescence, immunohistochemistry, direct, indirect, competitive or sandwich ELISA, to determine the presence and/or amount of the peptide isolated in biological samples isolated from individuals (*in vitro* diagnosis and/or monitoring of celiac disease), or as part of the protein from which it comes in food extracts.

A preferred method for detection and quantification of the peptide of the invention using the antibody or the cell of the invention is a sandwich enzyme immunoassay, for example, based on the use of a pair of antibodies of the invention specific for the peptide of the invention with affinity for two epitopes of the peptide that are far enough apart to allow the steric interaction of the two antibody molecules with the peptide simultaneously, one of the antibodies binds to a solid support, for example, but without limitation, a plastic plate or a PVDF membrane, and the second antibody is used as a label conjugated with an enzyme that catalyzes a colorimetric reaction (or a fluorochrome in fluorometric techniques), thus the sample to be studied is incubated with these reagents and the color intensity or fluorescence is measured at the end of the process, being it directly proportional to the amount of peptide of the invention present in the sample. Another preferred method for detection and quantification of the peptide of the invention is a competitive enzyme immunoassay similar to the previous one but in which the marker antibody is replaced by the synthetic peptide of the invention conjugated with an enzyme or fluorochrome, then it is incubated with the test sample and, in this case the amount of peptide in the same is inversely proportional to the color developed or the fluorescence emitted. Another preferred method for the detection of the peptide of the invention consists in carrying out an immunochromatographic technique using a similar process than the first method described in this paragraph, but one of the antibodies of the couple is adsorbed in the PVDF and second antibody of the couple with the corresponding marker is used as detection substance. The latter technique allows rapid qualitative determinations.
As used herein, the term "biological sample isolated" refers, but is not limited to tissues and/or biological fluids of an individual obtained by any method known to one skilled in the art that serve to such end. The biological sample can be a tissue or a biological fluid, is preferably a serum sample, feces or intestinal aspiration, and more preferably a duodenal-jejunal fluid sample. The sample may be taken from non-human mammals, such as, but not limited to, rodents, ruminants, felines or canines, or more preferably a human.
Another aspect of the invention relates to the use of the peptide, antibody or cell of the invention for the *in vitro* diagnosis and/or monitoring of celiac disease in an individual. In this regard, another aspect of the disclosure relates to an *in vitro* method for the diagnosis and/or monitoring of celiac disease in an individual comprising:
a. detection and/or quantification in a biological sample isolated, preferably in a sample of feces or intestinal aspiration, more preferably in a sample of duodenal-jejunal fluid, of the peptide of the invention (presence of the peptide of the invention in said sample is indicative of a celiac phenotype), which may be carried out, for example but not limited to, using the antibody or the cell of the invention in the methods described above for detection and/or quantification of the peptide of the invention, or by gel electrophoresis, NMR or any other diagnostic imaging technique or by chromatographic or mass spectrometry techniques; or
b. detection and/or quantification in a biological sample isolated, preferably in a serum sample, immunological activities generated against the peptide of the invention by the humoral immune system (detection and/or quantification of antibodies to the peptide of the invention) or cell (detection and/or quantification of specific T lymphocites against the peptide of the invention), which may be performed using the peptide of the invention, for example, but not limited to, the methods described below.
Specific antibodies against the peptide of the invention in celiac disease are mainly of IgG and IgA isotypes separately, or both IgG+IgA. In this case, the antibodies against the peptide of the invention constitute a variant of antigliadin antibodies and provide the specificity that these markers are lacking of. Antigliadin antibodies (AGA) have the historical importance of being the first useful serological tool in the diagnosis of celiac disease. The appearance of antibodies to dietary factors in celiac disease has been known since the sixties of the last century. The AGA are directed against antigenic determinants of α-gliadin highly conserved and shared with the other fractions (gliadins β, γ and ω). However, these antibodies are not specific for celiac disease and can be found in other diseases and even in healthy individuals. By contrast, the antibodies against the peptide of the invention are those antigliadin, antisecalin, antiglutenin and antihordein antibodies with an affinity for gliadin, secalin, hordein and glutenin fragments specifically processed in the intestine of patients with celiac disease. Therefore, the detection and/or quantification of these antibodies in an isolated biological sample constitute an effective method for *in vitro* diagnosis and/or monitoring of celiac disease. The detection and/or quantification of these antibodies against the peptide of the invention, preferably against its variants deamidated by tTG, can be performed using the peptide of the invention in various immunological techniques such as, but not limited to:
- Immunochromatographic techniques, in which the peptide of the invention is adsorbed to nitrocellulose membrane strips, PVDF or similar materials and, through a lateral flow, the biological sample to be tested contacts with said peptide adsorbed. The specific antibodies that can be present in the sample are bound to the peptide adsorbed and the rest are dragged. These antibodies can be visualized by, for example, but not limited to, the reaction with anti-immunoglobulins antibodies (anti-lgA or anti-IgG) or any other substance capable of specifically binding to immunoglobulins (such as protein A or protein G), which preferably in turn would be combined with a coloured marker or a marker capable of generating colour, for example, but not limited to, colloidal gold. The reagents would be found set in different layers of the strip and be moved by the flow of the sample itself, so that this method is preferably performed in one step. This technique is qualitative, with the advantage of speed and no need for instrumentation for evaluation.
- Immunoassays (ELISA). In this case, the adsorption of the peptide of the invention is performed on a plastic, preferably as a multi-well plate, on which a reaction similar to that described in the previous paragraph is developed, although it is a semiquantitative technique so anti-Ig antibodies conjugated with enzymes are preferably used. Enzymes catalyze a colorimetric reaction after the addition of a chemical substrate that changes its color by the action of the enzyme. The amount of color generated is directly proportional to the quantity of specific antibodies present in the biological sample. A variant would be competitive techniques that evaluate the ability of the biological sample to inhibit the binding of a known amount of labeled peptide of the invention to antibodies against the peptide of the invention bound to the solid surface.
- Immunofluorometric techniques. These techniques are similar to ELISA in the main, but using a fluorescent substance as a marker for the secondary antibody. In cases where variations of polarization are used it is not necessary to fix the antigen to the solid surface. In this kind of techniques competitive variants are also used. Fluorochrome labeling increases the sensitivity of the technique versus enzyme immunoassays. Polystyrene beads can also be used as support for the antigen-antibody reaction and taking readings using a flow cytometer, which also allow to obtain a multiplex test whether different microspheres for each antigen are available, detecting the presence of other antigens in addition to the peptide of the invention.

Alternatively, for the *in vitro* diagnosis and/or monitoring of celiac disease the presence of T lymphocites specific for the peptide of the invention may be detected and/or quantified in the biological sample isolated by, for example, but without limitation, biological testing based on mononuclear cells from the individual, and preferably obtained from peripheral blood or isolated from the intestinal mucosa, and stimulated *in vitro* with the peptide of the invention. The response may be quantified by, for example, but not limited to, proliferation assays, blastogenesis, production of soluble substances or expression of cell membrane markers, and the results obtained from de analysis of the biological sample may be compared with positive controls, for example, using stimuli by T cell mitogens, as phytohemagglutinin, and negative controls, such as basal unstimulated cultures or with an innocuous stimulus. This method for the diagnosis and/or monitoring of celiac disease could replace gluten challenge *in vivo* tests because, as this test consists of *ex vivo* stimulation of lymphocytes isolated from the biological sample, would provide the advantage of not exposing the individual to a substance potentially harmful to his body when performing diagnosis and/or monitoring confirmation, and moreover this method would be more specific in determining sensitization to a substance which is specifically generated in the intestine of celiac patients and not in other pathologies.
The presence of an immune response, humoral or cellular, against the peptide of the invention relates to that peptides from gliadin, secalin, hordein or glutenin have passed the epithelial barrier of the intestinal mucosa and have been able to generate an adaptive response frompatient's immune system (immunologic memory) or to the presence of the peptide of the invention in the body of the individual from which the sample analyzed proceeds, which is indicative of a celiac intestinal phenotype in which the activity of an intestinal flora specific of celiac patients is included.

Another aspect of the disclosure relates to a method for *in vitro* diagnosis of celiac disease in an individual comprising:
a. the detection of proteolytic activity in an isolated biological sample, preferably from intestine, which leads to the formation of the peptide of the invention from gliadin, secalin, hordein or glutenin. The presence of the proteolytic activity in the tested sample is indicative of a celiac phenotype.

This could be done by the use of, for example but not limited to, the peptide of the invention modified with at least one chromogenic, fluorigenic or luminescent substrate. Incubation of the modified peptide with the isolated biological sample, preferably from the intestine, produces colour only in the case of celiac subjects, due to the presence of specific enzymes associated with their intestinal flora that are able to release by enzymatic digestion the peptide the invention.

The term "diagnosis" refers to determining the absence or presence of celiac disease in an individual. The term "monitoring" refers to analyzing the course or progression of celiac disease in an individual, preferably when the individual has been previously diagnosed with the disease, more preferably when the individual is subjected to a therapeutic treatment, and even more preferably when that individual is following a gluten-free diet.

Another aspect of the invention relates to the use of probiotic compounds that modify the intestinal bacterial flora responsible for the protease activity which hydrolyzes gliadin, secalin, hordein or glutenin generating the peptide of the invention, for the manufacture of a medicament for the treatment and/or prevention of celiac disease. Another aspect of the invention relates to the use of antibiotics that inhibit, partially or totally, the growth of the intestinal bacterial flora responsible for said protease activity, for the manufacture of a medicament for the treatment and/or prevention of celiac disease.

Another aspect of the invention relates to the use of the peptide, the nucleotide sequence, the antibody, the cell or the composition of the invention for the manufacture of a medicament, or alternatively, the peptide, the nucleotide sequence, the antibody, the cell or the composition of the invention for use as a medicine, hereafter "medicine of the invention".

The "medicine" to which the present invention relates may be for human or veterinary use. The "medicine for human use" means any substance or combination of substances presented as having properties for treating or preventing disease in human or that can be used in humans or administered to human in order to restore, correct or modify physiological functions by applying a pharmacological, immunological or metabolic action, or to make a medical diagnosis. The "medicine for veterinary use" means any substance or combination of substances presented for treating or preventing disease in animals or which may be administered to animals in order to restore, correct or modify physiological functions by applying a pharmacological, immunological or metabolic action, or to make a veterinary diagnosis.

The term "treatment", as understood herein, refers to combat the effects caused by celiac disease in a subject (preferably mammal, and more preferably human) which includes:
(i) inhibiting the disease or pathological condition, i.e., stopping its development;
(ii) alleviating the disease or the condition, i.e., causing regression of the disease or the condition or its symptoms, or
(iii) stabilizing disease or pathological condition.
The term "prevention" as understood herein, consists on prevent the onset of disease, that is, prevent the disease or pathological condition in a subject (preferably mammal, more and preferably human) to occur, in particular when said subject is predisposed to the pathological condition, but still has not been diagnosed as having it.

The term "vaccine" refers to an epitope or antigenic preparation used to elicit an immune response against one or several antigens. They are preparations from antigens or epitopes that, once inside the body, cause the immune response by producing antibodies and produce immunological memory by generating permanent or transient immunity. The vaccine of the invention may be administered to the subject once or several times (initial and subsequent administrations), depending o n an individual's ability to produce an immune response in response to the administration of the vaccine.

Another aspect of the invention relates to a kit, hereinafter "first kit of the invention", comprising the peptide of the invention. Another aspect of the invention relates to a kit, hereinafter "second kit of the invention", comprising the antibody or the cell of the invention.

The first and second kit of the invention may further comprise, without limitation, conjugated or free primary antibodies, peptides, buffers, conjugated secondary antibodies, conjugated streptavidin, protein or peptide standards, agents for pollution prevention, marker compounds, as for example, but not limited to, fluorochromes, etc. Moreover, the first and second kit of the invention may include all necessary brackets and containers for implementation and optimization. The first and second kit of the invention may additionally contain other proteins or peptides which act as positive and negative controls. Preferably these kits further comprise instructions for performing the *in vitro* diagnosis and/or monitoring of celiac disease in an individual, or to detect and/or quantify the peptide of the invention, preferably in food.

Optionally, the peptide, the antibody or the cell of the invention are labeled or immobilized in the kits of the invention. Preferably, they are marked with a label selected from the list comprising: a radioisotope, a fluorescent or luminescent label, an antibody, an antibody fragment, an affinity tag, an enzyme or an enzymatic substrate. More preferably, the peptide, the antibody or the cell of the invention are immobilized in the kits of the invention. The term "immobilized" as used herein, refers to the peptide, the antibody or the cell of the invention may be attached to a support without losing its activity. Preferably, the support may be the surface of a matrix (for example a matrix of nylon), a microtiter plate (for example a 96 well plate) or similar plastic support, or beads (spheres, for example, spheres of agarose or small microspheres made of biodegradable superparamagnetic matrixes).
Another aspect of the invention relates to the use of the second kit of the invention for the detection and/or quantification of the peptide of the invention. In a preferred embodiment, the detection and/or quantification of the peptide is performed in foods. Another aspect of the invention relates to the use of the first or second kit of the invention for the *in vitro* diagnosis and/or monitoring of celiac disease in an individual.

### FIGURE DESCRIPTION

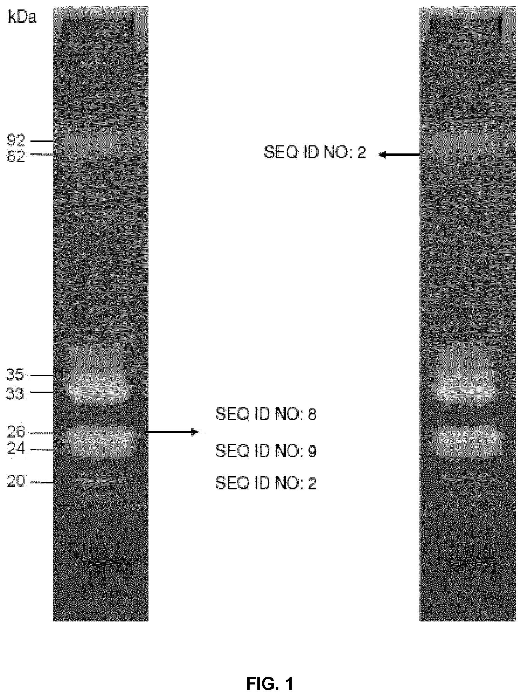

**Fig 1****. Shows the identification of three gliadin peptides** by independent zymograms with gliadin from two untreated celiac patients.

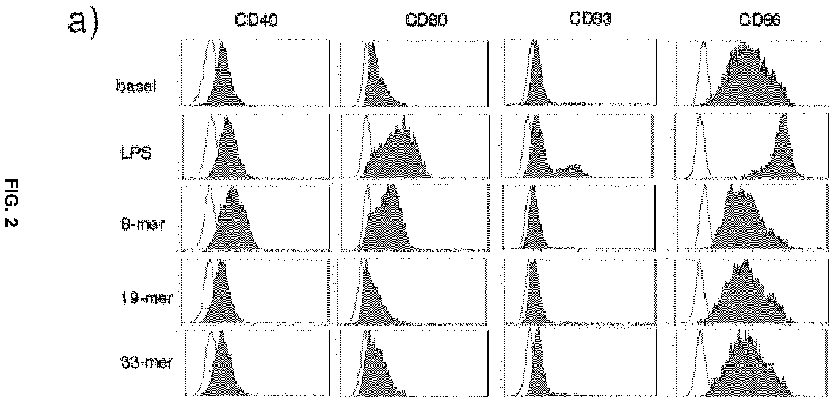

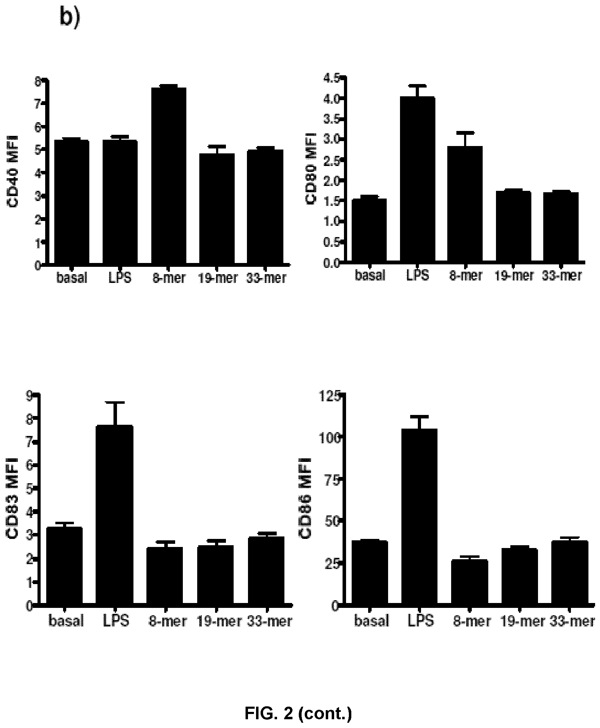

**Fig 2****. (a) Shows the representative histograms obtained by flow cytometry in dendritic cells derived from monocytes obtained from peripheral blood samples, ABO compatible, from healthy donors, stimulated** for 48 hours with lipopolysaccharide (LPS, 1 mg/mL), or with the gluten peptides 8-mer (SEQ ID NO: 2, 100 µg/mL), 19-mer (SEQ ID NO: 4, 100 µg/mL) and 33-mer (SEQ ID NO: 5, 100 µg/mL), and after basal conditions (internal control). Shadowed histograms represent the expression of the co-stimulator markers (CD40, CD80, CD86) or activators (CD83) in dendritic cells. Empty histograms represent isotype controls. This experiment is representative of three independent experiments; each of them is performed by triplicate. (b) It shows median fluorescence intensity (MFI) of a simple experiment performed by triplicate, representative of three independent experiments with similar results.

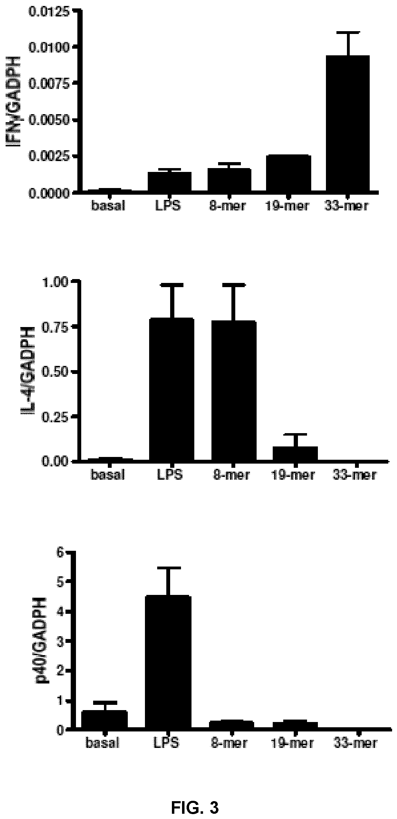

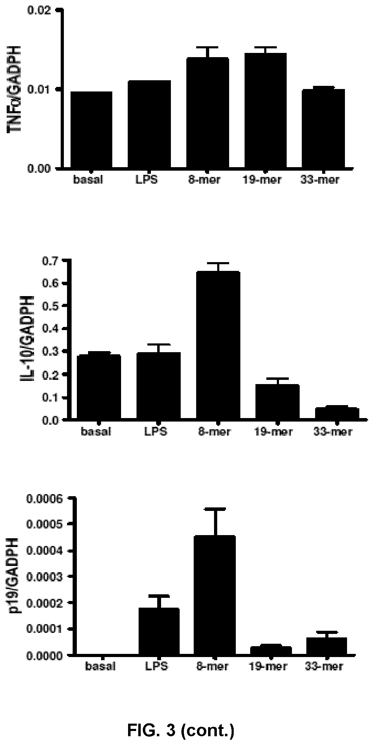

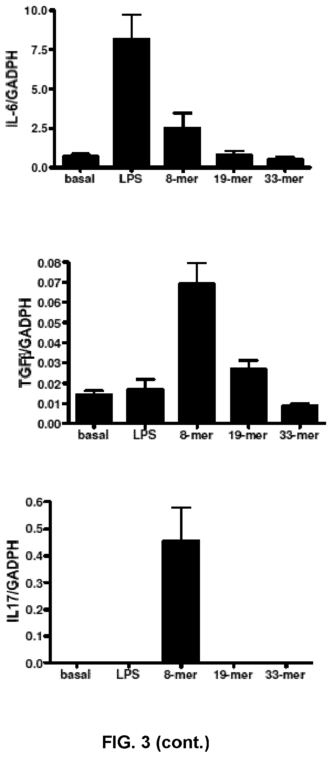

**Fig 3****. Shows RNA expression, in arbitrary units (AU) of IFNy, IL-4, IL-12p40, TNFα, IL-10, IL23p19, IL-6, TGFβ and IL-17 in dendritic cells derived from monocytes obtained from peripheral blood samples, ABO compatible, from healthy donors, stimulated** for 48 hours with lipopolysaccharide (LPS, 1mg/ml), or gluten peptides 8-mer (SEQ ID NO: 2, 100 µg/ml) 19-mer (SEQ ID NO: 4, 100 µg/ml) and 33-mer (SEQ ID NO: 5, 100 µg/ml), and after basal conditions (internal control). The mean and standard deviation of three independent experiments are shown.

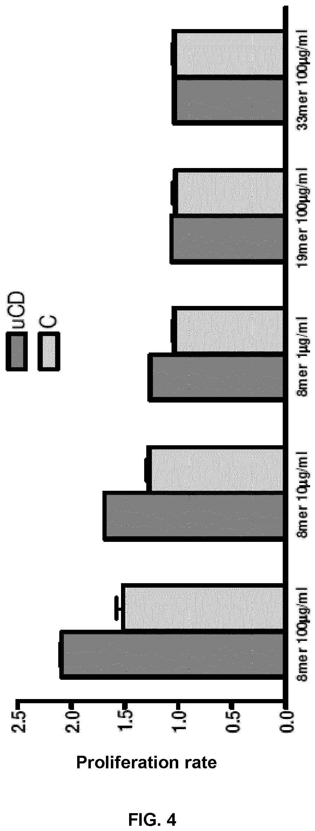

**Fig 4****. Shows the proliferation rate of mononuclear cells from peripheral blood enriched in T-cells after being cultured with dendritic cells from control subjects (C) or untreated celiac patients (uCD), previously stimulated** with the gluten peptides 8-mer (SEQ ID NO: 2, 100 µg/ml, 10 µg/ml and 1 µg/ml), 19-mer (SEQ ID NO: 4, 100 µg/ml) and 33-mer (SEQ ID NO: 5, 100 µg/ml) based on the basal proliferation induced by dendritic cells cultured in medium without stimulus and co-cultured with mononuclear cells from peripheral blood enriched with autologous T cells. The mean and standard deviation of an experiment performed in triplicate are shown, and which is representative of three independent experiments with similar results. Each experiment was performed with an untreated celiac patient with genotype HLA-DQ2⁺ and parallel to a healthy non-celiac individual HLA-DQ2⁻as a control (C).

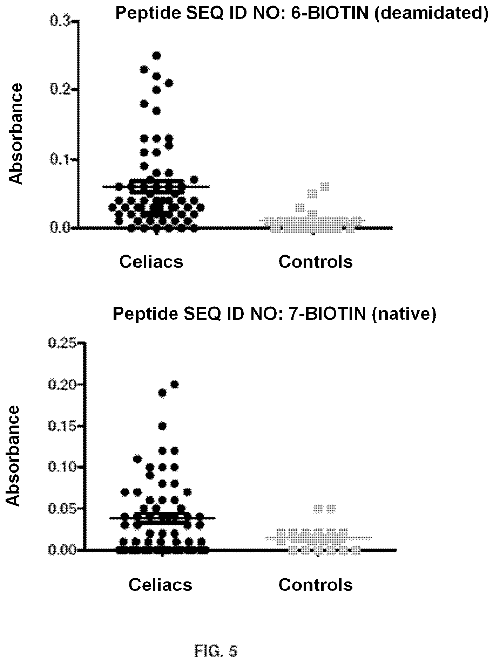

**Fig 5****. Shows the levels of anti-peptide 8-mer IgA antibodies (a) anti-deaminated peptide 8-mer (SEQ ID NO: 6) and (b) anti-peptide 8-mer native/unmodified (SEQ ID NO: 7), both labeled with biotin,** in sera from celiac patients and non-celiac controls.

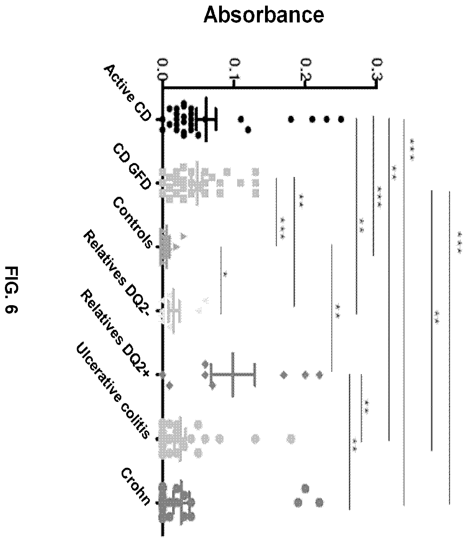

**Fig 6****. Shows the levels of anti- deamidated peptide 8-mer IgA antibodies in serum samples from patients with active celiac disease (CD), celiac patients on gluten-free diet (CD GFD), non-celiac controls, healthy relatives of celiac patients HLA-DQ2⁺, healthy relatives HLA-DQ2⁻, and patients with inflammatory bowel disease (ulcerative colitis or Crohn's disease).** Asterisks indicate statistically significant levels: (*) p <0.05, (**) p <0.005, (***) p <0.0005.

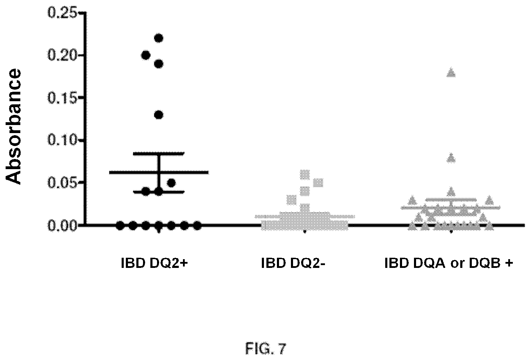

**Fig 7****. Shows the levels of anti- deaminated peptide 8-mer IgA antibodies in serum samples of patients with Inflammatory Bowel Disease (IBD)** according to the expression of HLA-DQ2 (HLA-DQ2⁺, HLA-DQ2⁻, and HLA-DQ2⁻ but positive for one of its chains, DQA or DQB).

### EXAMPLES

The invention will be illustrated by tests performed by the inventors, which demonstrate the effectiveness of the peptide of the invention for the *in vitro* diagnosis and/or monitoring in celiac disease. These specific examples serve to illustrate the nature of the present invention and are included for illustrative purposes only so that they must not be interpreted as limitations to the invention claimed herein. Therefore, the examples described below illustrate the invention without limiting the scope thereof.

### EXAMPLE 1. Identification of immunogenic peptide 8-mer in cereals toxic for celiac patients.

This example shows how the peptide 8-mer from gliadin was isolated and its immunogenic activity determined, and how the sequence of the peptide 8-mer can be found in gluten proteins from immunotoxic cereals. Due to the precedents of a unique pattern of bacterial metalloproteases capable of degrading the gliadin in the duodenal mucosa of celiac patients, an activity that was undetectable in healthy individuals (Bernardo et al., 2009, Gut.; 58:886-887), the identification of the products of such degradation was undertaken. First, trypsinized samples from degradation bands of 26 kDa and 82 kDa extracted from the zymogram performed with biopsy samples from untreated celiac patients (Fig. 1) were analyzed by ion trap mass spectrometry. By this method, three peptides were identified, SEQ ID NO: 2, SEQ ID NO: 8 and SEQ ID NO: 9, whose sequences were analyzed with a non-redundant database of proteins and, using the Blast tool of the NCBI it was found that these three peptides were present in wheat (wild and cultivated), barley or rye species, and within these species, only in prolamins (as gliadins, hordeins and secalins) and glutenins families. As gliadin used in the zymograms came from wheat, prolamins sequences present in wheat species were considered and it was found that only one of the peptides (called 8-mer, with sequence SEQ ID NO: 2) was not susceptible to been generated by trypsinization and, therefore, their presence was due exclusively to the action of proteases from the duodenal extract of the celiac patient. Below, Table 1 shows the species and groups of proteins containing complete 8-mer, which is widely distributed in several species of cereals toxic for celiacs (barley, rye and wheat).

**Table 1. Distribution of peptide SEQ ID NO: 2 in sequences of cereals toxic for celiac: barley, rye and several wheat, latter both wild and cultivated.**

| **8-mer (SEQ ID NO:2)** | | |
|---|---|---|
| 76 sequences | | |
| **Groups of proteins** (number of sequences) | **Species** | |
| | **Scientific name** (number of sequences) | **Common name** |
| **Omega gliadins** (25) | *Triticum aestivum* (6) | Common wheat (bread wheat) |
| | *Triticum urartu* (4) | Wild wheat |
| | *Triticum monoccocum subsp. aegilopoides* (2) | Wild spelt |
| | *Triticum monoccocum* (5) | Spelt wheat |
| | *Triticum turgidum subsp. paleocolchicum* (1) | - |
| | *Lophopyrum elongatum* (3) | Tall wheatgrass |
| | *Triticum aestivum x Lophopyrum elongatum* (3) | |
| | *Aegilops tauschii* (1) | Wild goatgrass (wheat ancestor) |
| **Omega secalins** (44) | *Triticum aestivum x Secale cereale* (2) | Octaploid triticale |
| | *Triticum turgidum subsp. durum x Secale cereale* (4) | Hexaploid triticale |
| | *Secale cereale* (8) | Rye |
| | *Triticum aestivum* (29) | Common wheat (bread wheat) |
| **Hordeins** (6) | *Hordeum vulgare* | Barley |
| | *Hordeum vulgare subsp. vulgare* | Barley |
| **LMW glutenins** (1) | *Triticum aestivum* (1) | Common wheat (bread wheat) |

As discussed below, the tests that demonstrate the immunostimulatory activity of the new peptide identified, 8-mer, were: *in vitro* dendritic cells activation (Fig. 2 and 3), and stimulation of autologous T cells mediated by dendritic cells (Fig 4). This peptide was found to be immunogenic in all tested individuals (celiac patients and healthy non-celiac controls). Clinical interest is that said immunogenic peptide is demonstrated to be generated only by the action of bacterial proteases isolated from the intestine of celiac patients. Finding a similar protease activity in duodenal biopsies of 11 non-celiac individuals was unsuccessful.

The results that demonstrate the immunogenic effect of this new gluten peptide are shown below, and embodiments in which this peptide was used to test *in vitro* the immunogenic activity from peripheral blood of controls are provided (Fig. 2 , 3 and 4). The advantages of the 8-mer peptide over the rest of immunotoxic gluten peptides identified in their applications in the diagnosis and treatment of celiac disease, lie in the fact that it is a natural peptide, generated *in vivo* by the action of bacterial proteases in the intestine of celiac patients, which combined with genetic predisposition plays an important role in celiac disease.

### EXAMPLE 2. Method for in vitro diagnosis of celiac disease by the detection of anti 8-mer antibodies in serum samples.

This example shows the development of an ELISA type method to detect the presence of anti 8-mer antibodies in individuals suspected of suffering from celiac disease. For this, a method of indirect ELISA was developed in order to detect antibodies against peptide 8-mer from gliadin in serum samples. Four variants of the biotinylated antigen/peptide were used (Biomedal, S.L., Seville, Spain): native form FK-9-1 (SEQ ID NO: 7-BIOTIN, SEQ ID NO: 7 corresponds to SEQ ID NO: 2 with a lysine at the C-terminus) and deamidated form FK(BIO)-9-2 (SEQ ID NO: 6-BIOTIN, SEQ ID NO: 6 corresponds to SEQ ID NO: 3 with a lysine at the C-terminus), due to its possible modification by tTG, with biotinylation in the N- or C-terminus. A plate with streptavidin was coated with them, at a concentration of 1 µg/mL (in PBS + Tween 0.05% solution), with an optimal preincubation of 1 hour at room temperature. After addition of the serum samples at 1:25 dilution with incubation for 2 hours at 37 ° C, a secondary antibody (rabbit polyclonal anti- human IgA/HRP antibody from Dako) at 1:4000 dilution was used. The studied subjects, from which the samples belong to, included the following groups: active celiac patients (CD activity), celiac patients in gluten-free diet (GFD CD), healthy controls, healthy relatives DQ2⁻ and healthy relatives DQ2⁺.

It was proved that the sera of celiac patients contained antibodies against peptide 8-mer from gliadin. From the four variants of peptide used in the standardization of the ELISA technique, optimal results were only obtained with the 2 peptides biotinylated at the C-terminus.

Sera from active celiac patients had higher levels of antibodies against the peptide 8-mer from gliadin than samples of the control group, although statistically significant differences were only observed in the case of deamidated peptide (p<0.0001) (Fig. 5).

Regarding the deamidated peptide significant differences are observed when comparing antibody levels in active celiac patients group and in the control group (p=0.0003) and between celiac patients in gluten-free diet (GFD CD) and healthy relatives (p=0.0006) (Fig. 6).

### EXAMPLE 3. Method for in vitro diagnosis of celiac disease, not detected by other methods, in patients with inflammatory bowel disease by the detection of anti 8-mer antibodies in serum samples.

This example shows how individuals with inflammatory bowel disease (IBD), which had not been previously diagnosed as celiac by conventional serology (tissue transglutaminase antibodies) or intestinal endoscopy, can be identified as celiacs when anti 8-mer antibodies appeared in serum.

In a group of 56 patients with IBD who did not have positive serology or intestinal biopsies for the detection of celiac disease the serum level of antibodies that recognize the 8-mer peptide was studied by the ELISA assay described in Example 2. Two samples of patients with ulcerative colitis and three of patients with Crohn's disease were so identified, which had levels of anti-peptide 8-mer antibodies similar to those obtained for celiac patients (Fig. 6). In this group of patients with IBD with elevated anti 8-mer antibodies, a study of genetic markers of celiac disease (HLA-DQ2) was carried out. The results are shown in the following table:

**Table 2. Results from the study of the celiac disease genetic markers (HLA-DQ2) in patients with IBD that present elevated levels of anti 8-mer antibodies.**

| **Patient** | **Diagnosis** | **DQA** | **DQB** |
|---|---|---|---|
| 1 | Ulcerative colitis | - | + |
| 2 | Ulcerative colitis | + | + |
| 3 | Crohn's disease | + | + |
| 4 | Crohn's disease | + | + |
| 5 | Crohn's disease | + | + |

Next, the same study of celiac disease genetic markers (DQ2) was performed on the remaining IBD patients whose samples were analyzed for anti-peptide 8-mer antibodies. Genetic markers of the 56 patients diagnosed with IBD that were detected are shown in the following table:

**Table 3. Results from the study of celiac disease genetic markers (HLA-DQ2) in all IBD patients whose samples were analyzed for anti peptide 8-mer antibodies.**

| **Number of patients** | **DQA** | **DQB** |
|---|---|---|
| 13 | + | + |
| 20 | - | - |
| 11 | + | - |
| 12 | - | + |

Fig. 7 shows the levels of anti deamidated 8-mer antibodies in IBD patients according to the expression of HLA-DQ2. These results demonstrate that individuals with IBD and high levels of anti deamidated peptide 8-mer antibodies also present positive celiac disease genetic markers (HLA-DQ2), indicating a high probability of being celiac. Therefore, the detection of anti 8-mer antibodies, preferably deamidated, allows identifying groups of celiac individuals that are not detectable by other diagnostic techniques.

### EXAMPLE 4. Immunogenic activity of 8-mer peptide in the cell immune system.

This example shows how 8-mer peptide can be used for the diagnosis of celiac disease by stimulation assays in autologous culture of dendritic cells and T lymphocytes belonging to celiac patients HLA-DQ2⁺ and non-celiac HLA-DQ2⁻controls.

Monocyte-derived dendritic cells serve as antigen presenting cells. Stimulation experiments carried out in the present invention included a positive proliferation control (T lymphocytes cultured in the presence of monocyte-derived dendritic cells pre-activated in the presence of lipopolysaccharide, LPS) and two negative controls: CD14- fraction enriched with cultured T lymphocytes i) in basal medium and ii) in the presence of monocyte-derived dendritic cells unstimulated.

Mononuclear cells from ABO-compatible peripheral blood were isolated by centrifugation on a density gradient, and magnetic beads with anti-CD14 antibodies to separate CD14+ monocytes, that were induced for 6 days to differentiate into immature dendritic cells by IL-4 and GM-CSF (confirmed by flow cytometry) were isolated too. These cells were first stimulated for 48 hours with the 8-mer peptide (100 µg/mL, 10 µg/mL, 1 µg/mL) and other known gliadin peptides: 19-mer (100 µg/mL) and 33-mer (100 µg/mL) and then a co-culture with autologous T lymphocytes was performed (population CD14- obtained above), in which dendritic cells act as antigen presenting cells. As negative and positive controls of stimulation, a CD14- cell culture without dendritic cells (basal), and CD14- cells stimulated with lipopolysaccharide (LPS) were used respectively. After 4 days, the cell proliferation rate was calculated according to the absorbance ratio of stimulated/unstimulated dendritic cells in co-culture with autologous T lymphocytes.

Stimulation of dendritic cells in culture from healthy control samples with 8-mer peptide induced phenotypic maturation of these cells, which was manifested by increased membrane expression of CD40 and CD80 markers, but not of CD83 or CD86. By contrast, peptides 19- and 33-mer did not affect the expression of these molecules (Fig. 2a, 2b). Stimulation with gliadin peptides was associated with a differential expression of cytokine mRNA profile (Fig. 3), with increased expression of IFNγ (8-mer: x14.2 times, 19-mer: x22.3 times, 33-mer: x85.8 times) and to a much lesser extent, TNFα (8-mer: x1.45 times, 19-mer: x1.51 times, 33-mer: x1.03 times). Major differences were observed with respect to IL-10, while 19- and 33-mer reduced their expression (19-mer: x0.16 times, 33-mer: x0.5 times), peptide 8-mer had the opposite effect (increased x2.3 times), and something similar happened with the IL-4 that increased with 8-mer (x79 times), moderately with 19-mer (x7.7 times), and was inhibited with 33-mer (x0.001 times). By contrast, the expression of IL-12p40 decreased slightly with 8- and 19-mer (x0.4 times both), was inhibited with 33-mer, and in any case IL-12p35 or IL-21 was detected. Stimulation with 8-mer could follow a pathway of differential activation through a Th17 cytokine profile, as indicated by the increase in IL-23p19 (8-mer: x45.3 times, 19-mer: x2.7 times; 33-mer: x6.2 times), and in other related cytokines such as IL-6 (8-mer: x3.6 times, 19-mer: x1.2 times, 33-mer: x0.7 times), and TGFβ (8-mer: x4.8 times, 19-mer: x1.9 times, 33-mer: x0.6 times).

After stimulation with peptide 8-mer, the dendritic cells in co-culture induced a proliferative response of autologous T lymphocytes in a dose dependent way, with cells from celiac patients HLA-DQ2⁺ as well as with control non-celiac individuals HLA-DQ2⁻ (100 µg/ml: proliferation rate 2.09 ± 0.029 in celiac individuals, and 1.51 ± 0.108 in non-celiac individuals; 10 µg/ml: proliferation rate 1.69 ± 0.005 in celiac individuals, and 1.28 ± 0.034 in non-celiac individuals; 1 µg/ml: proliferation rate 1.27 ± 0.005 in celiac individuals, and 1.04 ± 0.039 in non-celiac individuals) (Fig. 4). In all the cases studied (3 celiac patients DQ2⁺ and 3 control non-celiac individuals DQ2⁻) a response to peptide 8-mer was observed; however, it was more intense in celiac patients, as indicated by the increased rates of proliferation in all cases for the same concentration of stimulus. By contrast, dendritic cells stimulated with peptides 19- and 33-mer did not induce proliferation in autologous T lymphocytes in any of the samples (19-mer: proliferation rate 1.06 ± 0.011 in celiac patients, and 1.03 ± 0.039 in non-celiac individuals, 33-mer: proliferation rate 1.04 ± 0.005 in celiac patients, and 1.04 ± 0.018 in non-celiac individuals) (Fig. 4).

### EXAMPLE 5. Development of a new monoclonal antibody for the detection of the peptide 8-mer.

The present example shows a strategy for obtaining monoclonal antibodies which recognize the peptide 8-mer from gliadin and which can be used, for example, to determine the presence of gluten in food.

A encoding sequence of 6 tandem copies of the 8-mer peptide was merged into an expression vector CASCADE, pALEX1a (Biomedal S.L., Seville, Spain), by the 5' end to a polyhistidine tag (6Xhistag) and by the 3' end to the DNA encoding a coadjuvant protein as a fragment of heat shock protein HSP70 from *Trypanosoma cruzi.* Using a strain of *E. coli* REG12 (Biomedal, S.L.) the expression of the fusion protein was induced with salicylate and subsequently it was purified by ion affinity chromatography (IMAC) to obtain a purity degree greater than 95%.

Two monoclonal antibodies against peptide 8-mer from gliadin was generated according to the standard method, with some specific modifications. In summary, two groups of Balb / c mice, obtained from IFFA-CREDO (Saint Germain sur I' Arbesle, France) were immunized twice subcutaneously at a dose of 0.025 mg of 8-mer-T-HSP70 or purified 8mer-X2-HSP70 recombinant protein as immunogen. Two weeks after the last immunization, a third dose of the fusion protein was inoculated intravenously into the mice in each group. All immunizations were carried out without adjuvant. Antibody titers were assessed by ELISA against biotinylated peptides 8-mer described in Example 2, using plates coated with streptavidin on day 4 after the last immunization. Immunized mice were culled, and spleens were removed for its use as a source of cells for fusion with myeloma cells SP2. Only spleen cells from immunized mice were fused with myeloma cells previously prepared and grown in RPMI medium supplemented with fetal bovine serum and 20% of aminopterin-thymidine containing hypoxanthine because hypoxanthine-aminopterin-thymidine medium only allows fused cells to survive in culture. The fused cells were distributed into 96-well plates with medium supplemented with aminopterin and containing the feeder cells derived from peritoneal saline washes from mouse.

The antibodies produced by the selected hybridomas were tested for their ability to react with epitopes from the gluten of wheat, barley and rye, on one hand, and maize and rice on the other hand by ELISA assay in which wells were coated with gluten from each species. Hybridomas which produced antibodies with more sensitive reactivity to gluten of wheat, barley and rye, and gave no reactivity in rice or maize, were selected.

With these antibodies direct, blocking or competitive ELISAs can be developed to detect the peptide of the invention, for example in food samples. The direct ELISA test can be carried out directly extracting gluten peptides from the food sample, immobilizing them in the plates and then using one of the antibodies developed in the present invention to react against 8-mer peptides from the immobilized sample. Development may be performed, for example, by the previous conjugation of the antibody to a peroxidase type enzyme, whose activity can be studied by measuring the amount of colored compound that accumulates after adding the appropriate chromogenic reagent.

## Claims

1. Isolated peptide with sequence SEQ ID NO: 1, SEQ ID NO: 2 or SEQ ID NO:3.

2. Peptide according to claim 1 which further comprises a chemical compound and/or a heterologous polypeptide bound to its N- and/or C-terminus.

3. Peptide according to claim 2 wherein the chemical compound is a biotin, chromogenic, fluorogenic or luminescent compound.

4. Isolated nucleotide sequence encoding for the peptide according to any one of claims 1 to 3.

5. Antibody against the peptide of claim 1.

6. Cell which expresses the antibody according to claim 5.

7. Composition comprising the peptide according to any of claims 1 to 3, the nucleotide sequence according to claim 4, the antibody according to claim 5 or the cell according to claim 6.

8. Use of the peptide according to any of claims 1 to 3 or the nucleotide sequence according to claim 4 for the *in vitro* identification or design of compounds or compositions for the *in vitro* diagnosis
of celiac disease.

9. Use of the antibody according to claim 5 or the cell according to claim 6 for the *in vitro* detection and/or quantification of the peptide according to claim 1.

10. Use of the antibody or the cell according to claim 9 in which the detection and/or quantification of the peptide is performed in food.

11. Use of the peptide according to any one of claims 1 to 3, the antibody according to claim 5 or the cell according to claim 6 for the *in vitro* diagnosis and/or monitoring of celiac disease in an individual.

12. Peptide according to any one of claims 1 to 3, the nucleotide sequence according to claim 4, the antibody according to claim 5, the cell according to claim 6 or the composition according to claim 7 for use as a medicine.

13. Peptide, nucleotide sequence, antibody, cell or composition for use according to claim 12, wherein the medicine is a vaccine.

14. Kit comprising the peptide according to any one of claims 1 to 3 and/or the antibody according to claim 6.

15. Use of the kit according to claim 14 for the *in vitro* detection and/or quantification of the peptide according to claim 1 and/or for the *in vitro* diagnosis and/or monitoring of celiac disease in an individual.

16. Use of the kit according to claim 15 wherein the detection and/or quantification of the peptide is performed in food and/or the *in vitro* diagnosis and/or monitoring of celiac disease is performed in a biological sample obtained from an individual.

## Patentansprüche

1. Isoliertes Peptid mit der Sequenz SEQ ID NO: 1, SEQ ID NO: 2 oder SEQ ID NO: 3.

2. Peptid nach Anspruch 1, das ferner eine chemische Verbindung und/oder ein heterologes Polypeptid umfasst, das an seinen N- und/oder C-Terminus gebunden ist.

3. Peptid nach Anspruch 2, wobei die chemische Verbindung eine Biotin-, chromogene, fluorgene oder lumineszierende Verbindung ist.

4. Isolierte Nukleotidsequenz, die für das Peptid nach einem der Ansprüche 1 bis 3 codiert.

5. Antikörper gegen das Peptid nach Anspruch 1.

6. Zelle, die den Antikörper nach Anspruch 5 exprimiert.

7. Zusammensetzung, umfassend das Peptid nach einem der Ansprüche 1 bis 3, die Nukleotidsequenz nach Anspruch 4, den Antikörper nach Anspruch 5 oder die Zelle nach Anspruch 6.

8. Verwendung des Peptids nach einem der Ansprüche 1 bis 3 oder der Nukleotidsequenz nach Anspruch 4 für die In-vitro-Identifizierung oder das Design von Verbindungen oder Zusammensetzungen für die In-vitro-Diagnose von Zöliakie.

9. Verwendung des Antikörpers nach Anspruch 5 oder der Zelle nach Anspruch 6 für den In-vitro-Nachweis und/oder die Quantifizierung des Peptids nach Anspruch 1.

10. Verwendung des Antikörpers oder der Zelle nach Anspruch 9, wobei der Nachweis und/oder die Quantifizierung des Peptids in Lebensmitteln ausgeführt wird.

11. Verwendung des Peptids nach einem der Ansprüche 1 bis 3, des Antikörpers nach Anspruch 5 oder der Zelle nach Anspruch 6 für die In-vitro-Diagnose und/oder Überwachung von Zöliakie in einem Individuum.

12. Peptid nach einem der Ansprüche 1 bis 3, die Nukleotidsequenz nach Anspruch 4, der Antikörper nach Anspruch 5, die Zelle nach Anspruch 6 oder die Zusammensetzung nach Anspruch 7 zur Verwendung als ein Medikament.

13. Peptid, Nukleotidsequenz, Antikörper, Zelle oder Zusammensetzung zur Verwendung nach Anspruch 12, wobei das Medikament ein Impfstoff ist.

14. Kit, umfassend das Peptid nach einem der Ansprüche 1 bis 3 und/oder den Antikörper nach Anspruch 6.

15. Verwendung des Kits nach Anspruch 14 für den In-vitro-Nachweis und/oder die Quantifizierung des Peptids nach Anspruch 1 und/oder für die In-vitro-Diagnose und/oder Überwachung von Zöliakie in einem Individuum.

16. Verwendung des Kits nach Anspruch 15, wobei der Nachweis und/oder die Quantifizierung des Peptids in Lebensmitteln ausgeführt wird und/oder die In-vitro-Diagnose und/oder Überwachung von Zöliakie in einer biologischen Probe ausgeführt wird, die von einem Individuum erhalten wird.

## Revendications

1. Peptide isolé avec la séquence SEQ ID NO : 1, SEQ ID NO : 2 ou SEQ ID NO : 3.

2. Peptide selon la revendication 1 qui comprend en outre un composé chimique et/ou une polypeptide hétérologue liés à sa terminaison N- et/ou C-.

3. Peptide selon la revendication 2 dans lequel le composé chimique est un composé de biotine, chromogénique, fluorogénique ou luminescent.

4. Séquence nucléotidique isolée codant le peptide selon l'une quelconque des revendications 1 à 3.

5. Anticorps contre le peptide selon la revendication 1.

6. Cellule qui exprime l'anticorps selon la revendication 5.

7. Composition comprenant le peptide selon l'une quelconque des revendications 1 à 3, la séquence nucléotidique selon la revendication 4, l'anticorps selon la revendication 5 ou la cellule selon la revendication 6.

8. Utilisation du peptide selon l'une quelconque des revendications 1 à 3 ou de la séquence nucléotidique selon la revendication 4 pour l'identification ou la conception *in vitro* de composés ou compositions pour le diagnostic *in vitro* de la maladie coeliaque.

9. Utilisation de l'anticorps selon la revendication 5 ou de la cellule selon la revendication 6 pour la détection et/ou la quantification *in vitro* du peptide selon la revendication 1.

10. Utilisation de l'anticorps ou de la cellule selon la revendication 9 dans laquelle la détection et/ou la quantification du peptide sont réalisées dans des aliments.

11. Utilisation du peptide selon l'une quelconque des revendications 1 à 3, de l'anticorps selon la revendication 5 ou de la cellule selon la revendication 6 pour le diagnostic et/ou la surveillance *in vitro* de la maladie coeliaque chez un individu.

12. Peptide selon l'une quelconque des revendications 1 à 3, la séquence nucléotidique selon la revendication 4, l'anticorps selon la revendication 5, la cellule selon la revendication 6 ou la composition selon la revendication 7 pour leur utilisation comme médicament.

13. Peptide, séquence nucléotidique, anticorps, cellule ou composition en vue de leur utilisation selon la revendication 12, dans lesquels le médicament est un vaccin.

14. Kit comprenant le peptide selon l'une quelconque des revendications 1 à 3 et/ou l'anticorps selon la revendication 6.

15. Utilisation du kit selon la revendication 14 pour la détection et/ou quantification *in vitro* du peptide selon la revendication 1 et/ou pour le diagnostic et/ou la surveillance *in vitro* de la maladie coeliaque chez un individu.

16. Utilisation du kit selon la revendication 15 dans laquelle la détection et/ou la quantification du peptide sont réalisées dans des aliments et/ou le diagnostic et/ou la surveillance *in vitro* de la maladie coeliaque sont réalisés dans un échantillon biologique obtenu chez un individu.
